(19) Europäisches Patentamt
European Patent Office
Office européen des brevets

(11) Veröffentlichungsnummer: **0 187 209 B1**

# (12) EUROPÄISCHE PATENTSCHRIFT

(45) Veröffentlichungstag der Patentschrift: **08.01.92**

(51) Int. Cl.[5]: **C07D 501/18**, C07D 501/20

(21) Anmeldenummer: **85113276.1**

(22) Anmeldetag: **19.10.85**

## (54) Verfahren zur Herstellung von Cephalosporinestern.

(30) Priorität: **15.11.84 CH 5470/84**

(43) Veröffentlichungstag der Anmeldung:
**16.07.86 Patentblatt 86/29**

(45) Bekanntmachung des Hinweises auf die Patenterteilung:
**08.01.92 Patentblatt 92/02**

(84) Benannte Vertragsstaaten:
**AT BE CH DE FR GB IT LI NL**

(56) Entgegenhaltungen:
**EP-A- 53 077**
**FR-A- 2 389 632**

**SYNTHESIS, Nov 1972; pp. 591-598**

(73) Patentinhaber: **F. HOFFMANN-LA ROCHE AG**
**Postfach 3255**
**CH-4002 Basel(CH)**

(72) Erfinder: **Furlenmeier, André, Dr.**
**Wettsteinallee 119**
**CH-4058 Basel(CH)**

(74) Vertreter: **Martin, Björn et al**
**Grenzacherstrasse 124 Postfach 3255**
**CH-4002 Basel(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

In EP-A-53077 wird die Solubilisierung von Cephalosporinen mit Hilfe von bicyclischen Amidinen beschrieben.

Die vorliegende Erfindung betrifft ein Verfahren zur Herstellung von Cephalosporinestern der allgemeinen Formel

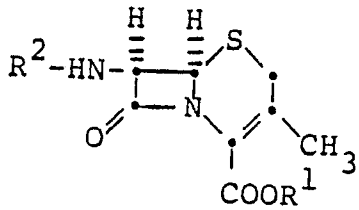

I

worin $R^2$ Wasserstoff oder eine Acylgruppe und $R^1$ eine leicht abhydrolysierbare Gruppe bedeutet, und von pharmazeutisch annehmbaren Säureadditionssalzen von basischen Verbindungen der Formel I, worin $R^2$ eine Acylgruppe bedeutet, und ist dadurch gekennzeichnet, dass man 7-Amino-3-methyl-3-cephem-4-carbonsäure (7-ADCA) in einem partiell chlorierten niederen Alkan mit einem bicyclischen Amidin der allgemeinen Formel

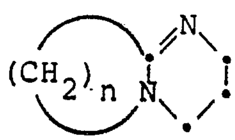

II

worin n die Zahl 3, 4 oder 5 bedeutet, und einem Halogenid der allgemeinen Formel

$X - R^1$ III

worin X Halogen bedeutet, und $R^1$ obige Bedeutung besitzt, umsetzt, die erhaltene Verbindung der Formel I, worin $R^2$ Wasserstoff bedeutet, erwünschtenfalls mit einem gegebenenfalls geschützten Acylierungsmittel behandelt, eine allenfalls vorhandene Schutzgruppe abspaltet und erwünschtenfalls eine erhaltene, basische Verbindung der Formel I, worin $R^2$ eine Acylgruppe bedeutet, in ein pharmazeutisch annehmbares Säureadditionssalz überführt.

Zur Herstellung von Verbindungen der Formel I, worin $R^2$ eine Acylgruppe bedeutet, wird der nach der Behandlung mit den Verbindungen II und III erhaltene Ester der allgemeinen Formel

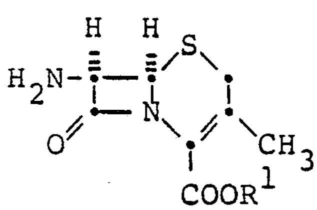

Ia

worin $R^1$ obige Bedeutung besitzt, vorzugsweise nicht isoliert, sondern im sogenannten "Eintopfverfahren" direkt durch Acylierung in die gewünschte Verbindung der allgemeinen Formel

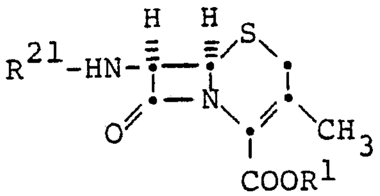

Ib

worin $R^1$ die obige Bedeutung besitzt und $R^{21}$ eine Acylgruppe bedeutet, übergeführt.

Der in der vorliegenden Beschreibung verwendete Ausdruck druck "Acylgruppe" bezeichnet Vorzugsweise von Carbonsäuren abgeleitete Acylgruppen, insbesondere in der Cephalosporin-Chemie für die 7-Aminogruppe verwendbare Acylgruppen. Als Beispiele dafür seien die folgenden Gruppen erwähnt: $Q-(A)_p-CO-$ und $Q-C(=NOR^3)CO-$, worin A niederes Alkylen, Q Aryl, Heteroaryl oder 5- bis 7-gliedriges Cycloalkyl, Cycloalkenyl, Heterocycloalkyl oder Heterocycloalkenyl, $R^3$ niederes Alkyl, niederes Alkenyl oder die Gruppe $-CH_2COOR^4$ oder $-C(CH_3)_2COOR^4$, die Gruppe $-COOR^4$ Carboxy oder eine leicht hydrolysierbare Estergruppe und p die Zahl 0 oder 1 bedeuten, wobei diese Gruppen beispielsweise durch Amino, Hydroxy, niederes Alkyl, niederes Alkoxy oder Halogen substituiert sein können. Beispiele derartiger Gruppen sind: Phenylacetyl, 2-Amino-2-phenylacetyl, 2-Amino-2-(4-hydroxyphenyl)acetyl, 2-Amino-2-(1,4-cyclohexadien-1-yl)acetyl, 2-(2-Amino-4-thiazolyl)acetyl, 2-(2--Furyl)-2-methoxyimino-acetyl, 2-(2-Amino-4-thiazolyl)-2-methoxyimino-acetyl, 2-(2-Amino-4-thiazolyl)-2-carboxymethoxyimino-acetyl und 2-(2-Amino-4-thiazolyl) -2-pivaloyloxymethoxycarbonylmethoxyimino-acetyl.

Der Ausdruck "leicht abhydrolysierbare Gruppe" bezeichnet vorzugsweise unter physiologischen Bedingungen abhydrolysierbare Gruppen, insbesondere niedere Alkanoyloxy-niedere Alkylgruppen, wie die Acetoxymethyl-, Pivaloyloxymethyl-, 1-Acetoxyäthyl- und die 1-Pivaloyloxyäthylgruppe, und niedere Alkoxycarbonyloxy-niedere Alkylgruppen, wie die Methoxycarbonyloxymethyl-, 1-Aethoxycarbonyloxyäthyl- und die 1-Isopropoxycarbonyloxyäthylgruppe. Der Ausdruck "nieder" bezeichnet Reste und Verbindungen mit höchstens 7, vorzugsweise höchstens 4 Kohlenstoffatomen. Der Ausdruck "Alkyl" und davon abgeleitete Ausdrücke, wie Alkoxy, Alkylen, Alkanoyl und Alkenyl, bezeichnen sowohl geradkettige als auch verzweigte Gruppen, wie Methyl, Aethyl, Isopropyl, t-Butyl und 2-Butyl, und die entsprechenden davon ableitbaren Gruppen.

Zur Durchführung des erfindungsgemässen Verfahrens kann die 7-ADCA in einem partiell chlorierten niederen Alkan, vorzugsweise in Methylenchlorid oder Chloroform, suspendiert werden, worauf man mit dem bicyclischen Amidin der Formel II als Salzbildner, vorzugsweise DBU, versetzt. Der Salzbildner soll dabei in stöchiometrischen Mengen oder, was bevorzugt wird, in geringem Unterschuss verwendet werden, weil überschüssige Zugabe zu ungewünschter $\Delta^2$-Isomerisierung am Cephalosporingerüst führt.

Das gebildete Salz der Verbindung der Formel II mit 7-ADCA wird der erfindungsgemässen Veresterung unterworfen, indem man ein Halogenid der Formel III, insbesondere ein Jodid, z.B. Pivaloyloxymethyljodid, zugibt. Zur Vermeidung von ungewünschter Gelbfärbung wird die Reaktion vorzugsweise unter Lichtausschluss durchgeführt.

Zur Herstellung der Endprodukte der Formel Ib kann der so erhaltene Ester der Formel Ia acyliert werden. Als Acylierungsmittel dienen verschiedene, den Acylrest $R^{21}$ abgebende Mittel. Im Acylierungsmittel allfällig vorhandene Aminogruppen sind in der Regel geschützt, wobei aliphatische Aminogruppen (z.B. wenn $R^{21}$ 2-Amino-2-phenylacetyl, 2-Amino-2-(4-hydroxyphenyl)acetyl, 2-Amino-2-(1,4-cyclohexadien-1-yl)-acetyl bedeutet) beispielsweise durch Trityl oder durch eine Gruppe der Formel $R^5R^6C=CR^7-$, worin $R^5$ niederes Alkanoyl, niederes Alkoxycarbonyl oder Benzoyl, $R^6$ Wasserstoff, niederes Alkyl, niederes Alkanoyl, niederes Alkoxycarbonyl oder Cyan und $R^7$ Wasserstoff oder niederes Alkyl bedeuten (z.B. 1-Methyl-2-methoxycarbonylvinyl, 1-Methyl-2-benzoylvinyl, 2,2-(Diäthoxycarbonyl)vinyl) und 1-Methyl-1-benzoylvinyl) und aromatische Aminogruppen (z.B. wenn $R^{21}$ 2-(2-Amino-4-thiazolyl)acetyloder 2-(2-Amino-4-thiazolyl)-2-methoxyiminoacetyl bedeutet) vorzugsweise durch sauer-hydrolytisch abspaltbare Gruppen, wie z.B. die t-Butoxycarbonyl- oder die Tritylgruppe, basisch-hydrolytisch abspaltbare Gruppen, wie z.B. die Trifluoracetylgruppe, oder durch eine Chlor-, Brom- oder Jodacetylgruppe, welche mit Thioharnstoff abgespalten werden kann, geschützt sind.

Als Acylierungsmittel kann die entsprechende Säure verwendet werden, wobei die Kondensation in diesem Fall in Gegenwart eines Kondensationsmittels, beispielsweise eines Carbodiimids, wie Dicyclohexylcarbobiimid, durchgeführt wird. Es können auch entsprechende Säurehalogenide eingesetzt werden. Vorzugsweise verwendet man jedoch Thioester der entsprechenden Säure, insbesondere die 2-Benzthiazolylthioester. Dies hat den Vorteil, dass aromatische Aminogruppen (z.B wenn $R^{21}$ 2-(2-Amino-4-thiazolyl)acetyl

oder 2-(2-Amino-4-thiazolyl)-2-methoxyimino-acetyl bedeutet) nicht geschützt werden müssen. Ein bevorzugter 2-Benzthiazolylthioester ist der 2-(2-Amino-4-thiazolyl-2-(Z)-methoxyimino-essigsäure -2-benzthiazolyl-thioester.

Nach der Acylierung werden allfällig vorhandene Aminoschutzgruppen entfernt: Sauer-hydrolytisch abspaltbare Schutzgruppen vorzugsweise mit Ameisensäure oder Trifluoressigsäure, basisch-hydrolytisch abspaltbare Schutzgruppen vorzugsweise mit wässriger Alkalilauge, die Chlor-, Brom- oder Jodacetylgruppe mit Thioharnstoff, und die Gruppe der Formel $R^5R^6C=CR^7$- biespielsweise durch Behandeln mit Wasser (gegebenenfalls im Gemisch mit einem mit Wasser mischbaren Lösungsmittel, wie Aceton, Tetrahydrofuran, Dioxan oder dergleichen) und einer Säure, wie Salz- oder Schwefelsäure, p-Toluolsulfonsäure oder einem schwefelsauren Ionenaustauscher.

Wie bereits erwähnt, wird das ganze Verfahren vorzugsweise im sogenannten "Eintopfverfahren", d.h. ohne Isolierung der erhaltenen Zwischenprodukte durchgeführt.

Die Reaktionstemperatur liegt jeweils vorzugsweise im Bereiche von etwa 0-40° C, wobei man vorzugsweise bei Raumtemperatur arbeitet.

Das erfindungsgemässe Verfahren bietet bedeutende produktionstechnische Vorteile, indem es in demselben Lösungsmittel glatt und in guter Ausbeute durchführbar ist. Es eignet sich insbesondere zur Herstellung von 7-[2-(2-Amino-4-thiazolyl)-2-(Z)-methoxyimino-acetamido] -3-methyl-3-cephem-4-carbonsäure-pivaloyloxymethylester, wobei man vorzugsweise DBU als Salzbildner, Methylenchlorid oder Chloroform als Lösungsmittel, Pivaloyloxymethyljodid als Halogenid der Formel III und 2-(2-Amino-4-thiazolyl)-2-(Z)-methoxyimino-essigsäure -2-benzthiazolyl-thioester als Acylierungsmittel verwendet und im "Eintopfverfahren" arbeitet, wobei die zusätzliche Stufe der Aminoschutzgruppenspaltung entfällt.

Die basischen Verbindungen der Formel Ib, d.h. Verbindungen der Formel Ib mit einer basischen Acylgruppe, können erwünschtenfalls in pharmazeutisch annehmbare Säureadditionssalze übergeführt werden, wobei deren Herstellung nach an sich bekannten und jedem Fachmann geläufigen Methoden erfolgen kann. Es kommen dabei sowohl Salze mit anorganischen als auch Salze mit organischen Säuren in Betracht, beispielsweise Hydrochloride, Hydrobromide, Sulfate, Nitrate, Citrate, Acetate, Maleate, Succinate, Methansulfonate, p-Toluolsulfonate und dergleichen. In einer speziellen Ausführungsform betrifft die vorliegende Erfindung die Herstellung des Hydrochlorides des 7-[2-(2-Amino-4-thiazolyl)-2-(Z)-methoxyimino-acetamido] -3-methyl-3-cephem-4-carbonsäure-pivaloyloxymethylesters.

Die Cephalosporinester der Formel Ib sind wertvolle Heilmittel für die Bekämpfung bzw. Prophylaxe von Infektionskrankheiten, insbesondere auf oralem Wege. Sie besitzen antibiotische, insbesondere antibakterielle Wirkung und ein breites Wirkungsspektrum gegen Gram-positive und Gram-negative Bakterien.

Das nachstehende Beispiel dient der näheren Erläuterung der vorliegenden Erfindung.

Beispiel

19,2 g (90 mM) 7-ADCA werden in 500 ml Methylenchlorid suspendiert, mit 13,2 ml (84 mM) DBU versetzt und 30 Minuten bei Raumtemperatur gerührt. Anschliessend werden 16 ml (100 mM) Pivaloyloxymethyljodid zugegeben. Man rührt 30 Minuten bei Raumtemperatur, versetzt mit 28 g (80 mM) 2-(2-Amino-4-thiazolyl)-2-(Z)-methoxyimino-essigsäure -2-benzthiazolyl-thioester und rührt unter Lichtausschluss 3,5 Stunden bei Raumtemperatur. Die erhaltene, trübe Lösung wird filtriert, dreimal mit je 1 1 Wasser gewaschen und im Vakuum bei 30° C eingedampft. Der gelbe Rückstand wird in 720 ml Isopropanol und 12 ml 25-proz. Salzsäure gelöst. Nach kurzer Zeit tritt Kristallisation ein. Unter Rühren trägt man dann innert 30 Minuten 860 ml Hexan ein. Das auskristallissierte rohe Hydrochlorid wird abgenutscht, im Vakuum getrocknet und aus Aethanol/Hexan umkristallisiert. Man erhält 30,8 g (70%) 7-[2-(2-Amino-4-thiazolyl)-2-(Z)-methoxyimino-acetamido] -3-methyl-3-cephem-4-carbonsäure-pivaloyloxymethylester-Hydrochlorid vom Schmelzpunkt 169-170° C (Zers.).

**Patentansprüche**

1. Verfahren zur Herstellung von Cephalosporinestern der allgemeinen Formel

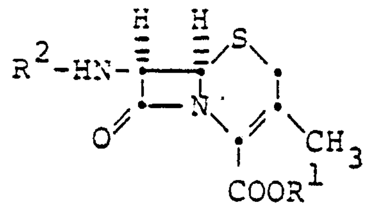

I

worin $R^2$ Wasserstoff oder eine Acylgruppe und $R^1$ eine leicht abhydrolysierbare Gruppe bedeuten, und von pharmazeutisch annehmbaren Säureadditionssalzen von basischen Verbindungen der Formel I, worin $R^2$ eine Acylgruppe bedeutet, dadurch gekennzeichnet, dass man 7-Amino-3-methyl-3-cephem-4-carbonsäure in einem partiell chlorierten niederen Alkan mit einer stöchiometrischen oder leicht unterschüssigen Menge eines bicyclischen Amidins der allgemeinen Formel

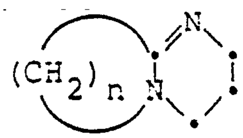

II

worin n die Zahl 3, 4 oder 5 bedeutet, und einem Halogenid der allgemeinen Formel

$X - R^1$ III

worin X Halogen bedeutet, und $R^1$ obige Bedeutung besitzt, umsetzt, die erhaltene Verbindung der Formel I, worin $R^2$ Wasserstoff bedeutet, erwünschtenfalls mit einem gegebenenfalls geschützten Acylierungsmittel behandelt, eine allenfalls vorhandene Schutzgruppe abspaltet und erwünschtenfalls eine erhaltene, basische Verbindung der Formel I, worin $R^2$ eine Acylgruppe bedeutet, in ein pharmazeutisch annehmbares Säureadditionssalz überführt.

2. Verfahren nach Anspruch 1, dadurch gekennzeichnet, dass man als partiell chloriertes niederes Alkan, Methylenchlorid oder Chloroform verwendet.

3. Verfahren nach Anspruch 1 oder 2, dadurch gekennzeichnet, dass man als bicyclisches Amidin der Formel II 1,8-Diazabicyclo[5.4.0]undec-7-en verwendet.

4. Verfahren nach einem der Ansprüche 1-3, dadurch gekennzeichnet, dass man das bicyclische Amidin in leichtem Unterschuss verwendet.

5. Verfahren nach einem der Ansprüche 1-4, dadurch gekennzeichnet, dass man als Verbindung der Formel III ein Pivaloyloxymethylhalogenid verwendet.

6. Verfahren nach einem der Ansprüche 1-5, dadurch gekennzeichnet, dass man eine Verbindung der Formel III verwendet, worin X Jod bedeutet.

7. Verfahren nach einem der Ansprüche 1-6, dadurch gekennzeichnet, dass man als Acylierungsmittel 2-(2-Amino-4-thiazolyl)-2-(Z)-methoxyimino-essigsäure -2-benzthiazolylthioester verwendet.

8. Verfahren nach einem der Ansprüche 1-7, dadurch gekennzeichnet, dass die Reaktionstemperatur jeweils zwischen 0° und 40°C liegt.

9. Verfahren nach einem der Ansprüche 1-8, dadurch gekennzeichnet, dass man die Reaktionsfolge in demselben Lösungsmittel und ohne Isolierung der Zwischenprodukte durchführt.

10. Verfahren nach einem der Ansprüche 1-9, dadurch gekennzeichnet, dass man 7-[2-(2-Amino-4-thiazolyl)-2-(Z)-methoxyimino-acetamido] -3-methyl-3-cephem-4-carbonsäure-pivaloyloxymethylester oder dessen Hydrochlorid herstellt.

## Claims

1. A process for the manufacture of cephalosporin esters of the general formula

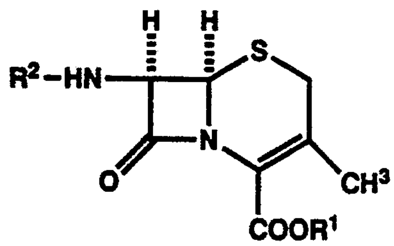

I

wherein $R^2$ signifies hydrogen or an acyl group and $R^1$ signifies a group which is readily removalbe by hydrolysis, and of pharmaceutically acceptable acid addition salts of basic compounds of formula I in which $R^2$ signifies an acyl group, characterized by reacting 7-amino-3-methyl-3-cephem-4-carboxylic acid in a partially chlorinated lower alkane with a bicyclic amidine of the general formula

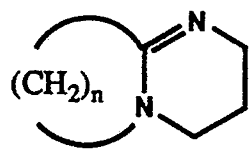

II

wherein n signifies the number 3, 4 or 5, and a halide of the general formula

$X-R^1$ III

wherein X signifies halogen and $R^1$ has the above significance, if desired, converting the resulting compound of formula I in which $R^2$ signifies hydrogen with an optionally protected acylating agent, cleaving off a protecting group which may be present and, if desired, converting a resulting basic compound of formula I in which $R^2$ signifies an acyl group into a pharmaceutically acceptable acid additon salt.

2. A process according to claim 1, characeterized in that methylene chloride or chloroform is used as the partially chlorinated lower alkane.

3. A process according to claim 1 to 2, characterized in that 1,8-diazabicyclo[5.4.0]undec-7-ene is used as the bicyclic amidine of formula II.

4. A process according to any one of claims 1-3, characterized in that the bicyclic amidine is used in a slight deficiency.

5. A process according to any one of claims 1-4, characterized in that a pivaloyloxymethyl halide is used as the compound of formula III.

6. A process according to any one of claims 1-5, characterized in that a compound of formula III in which X signifies iodine is used.

7. A process according to any one of claims 1-6, characterized in that 2-(2-amino-4-thiazolyl)2-(Z)-methoxy-imino-acetic acid 2-benzthiazolyl thioester is used as the acylating agent.

8. A process according to any one of claims 1-7, characterized in that the reaction temperature in each step lies between 0 ° C and 40 ° C.

9. A process according to any one of claims 1-8, characterized in that the reaction sequence is carried out in the same solvent and without isolating the intermediates.

10. A process according to any one of claims 1-9, characterized in that 7-[2-(2-amino-4-thiazolyl)-2-(Z)-methoxyimino-acetamido]-3-methyl-3-cephem-4-carboxylic acid pivaloyloxymethyl ester or its hydrochloride is manufactured.

**Revendications**

1. Procédé de préparation d'esters de céphalosporines de formule générale

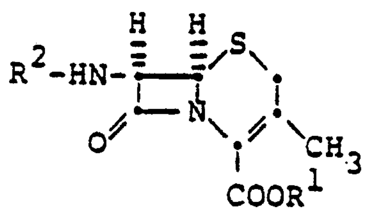

I

dans laquelle $R^2$ représente l'hydrogène ou un groupe acyle et $R^1$ représente un groupe facile à éliminer par hydrolyse,
et des sels des composés basiques de formule I dans laquelle $R^2$ représente un groupe acyle formés par addition avec des acides acceptables pour l'usage pharmaceutique, caractérisé en ce que l'on fait réagir l'acide 7-amino-3-méthyl-3-céphème-4-carboxylique, dans un alcane inférieur partiellement chloré, avec la quantité stoechiométrique ou un léger défaut d'une amidine bicyclique de formule générale

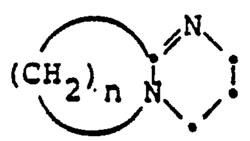

II

dans laquelle n est égal à 3, 4 ou 5,
et un halogénure de formule générale

$X - R^1$ III

dans laquelle X représente un halogène et $R^1$ a les significations indiquées ci-dessus, et si on le désire, on traite le composé obtenu, répondant à la formule I dans laquelle $R^2$ représente l'hydrogène, par un agent acylant éventuellement protégé, on élimine un groupe protecteur éventuellement présent et si on le désire, on convertit un composé basique ainsi obtenu, répondant à la formule I dans laquelle $R^2$ représente un groupe acyle, en un sel formé par addition avec un acide acceptable pour l'usage pharmaceutique.

2. Procédé selon la revendication 1, caractérisé en ce que l'on utilise en tant qu'alcane inférieur partiellement chloré le chlorure de méthylène ou le chloroforme.

3. Procédé selon la revendication 1 ou 2, caractérisé en ce que l'on utilise en tant qu'amidine bicyclique de formule II le 1,8-diazabicyclo[5.4.0]undéca-7-ène.

4. Procédé selon l'une des revendications 1 à 3, caractérisé en ce que l'on utilise l'amidine bicyclique en léger défaut.

5. Procédé selon l'une des revendications 1 à 4 caractérisé en ce que l'on utilise en tant que composé de

formule III un halogénure de pivaloyloxyméthyle.

6. Procédé selon une des revendications 1 à 5, caractérisé en ce que l'on utilise un composé de formule III dans laquelle X représente l'iode.

7. Procédé selon une des revendications 1 à 6, caractérisé en ce que l'on utilise en tant qu'agent acylant le thioester 2-benzothiazolylique de l'acide 2-(2-amino-4-thiazolyl)-2-(Z)-méthoxyimino-acétique.

8. Procédé selon une des revendications 1 à 7, caractérisé en ce que la température de réaction se situe dans chaque cas entre 0 et 40 °C.

9. Procédé selon une des revendications 1 à 8, caractérisé en ce que l'on procède à la séquence de réactions dans le même solvant et sans isolement des produits intermédiaires.

10. Procédé selon une des revendications 1 à 9 , caractérisé en ce que l'on prépare le 7-[2-(2-amino-4-thiazolyl)-2-(Z)-méthoxyimino-acétamido]-3-méthyl-3-céphème-4-carboxylate de pivaloyloxyméthyle ou son chlorhydrate.